(19) Europäisches Patentamt
European Patent Office
Office européen des brevets

(11) Veröffentlichungsnummer : **0 100 044**
**B1**

(12) # EUROPÄISCHE PATENTSCHRIFT

(45) Veröffentlichungstag der Patentschrift : **21.01.87**

(51) Int. Cl.[4] : **C 07 D285/08**, C 07 D417/12, A 01 N 43/82

(21) Anmeldenummer : **83107012.3**

(22) Anmeldetag : **18.07.83**

(54) **Substituierte 3-Trihalogenmethyl-1,2,4-thiadiazol-5-yl-oxyessigsäureamide, Verfahren zu ihrer Herstellung und ihre Verwendung als Herbizide.**

(30) Priorität : **28.07.82 DE 3228147**

(43) Veröffentlichungstag der Anmeldung : **08.02.84 Patentblatt 84/06**

(45) Bekanntmachung des Hinweises auf die Patenterteilung : **21.01.87 Patentblatt 87/04**

(84) Benannte Vertragsstaaten : **BE CH DE FR GB IT LI NL**

(56) Entgegenhaltungen :
**DE-A- 3 038 636**
**US-A- 4 228 290**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Förster, Heinz, Dr.**
**Am Eckbusch 47**
**D-5600 Wuppertal 1 (DE)**
Erfinder : **Klauke, Erich, Dr.**
**Eichendorffweg 8**
**D-5063 Odenthal (DE)**
Erfinder : **Eue, Ludwig, Dr.**
**Paul-Klee-Strasse 36**
**D-5090 Leverkusen 1 (DE)**
Erfinder : **Schmidt,Robert R., Dr.**
**Im Waldwinkel 110**
**D-5060 Bergisch-Gladbach 2 (DE)**
Erfinder : **Lürssen, Klaus, Dr.**
**August-Kierspel-Strasse 89**
**D-5060 Bergisch-Gladbach 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 100 044 B1

**Beschreibung**

Die Erfindung betrifft neue substituierte, fluorhaltige 3-Trihalogenmethyl-1,2,4-thiadiazol-5-yl-oxy-essigsäureamide, ein Verfahren zu ihrer Herstellung und ihre Verwendung als Herbizide.

Es ist bereits bekannt, daß bestimmte Azolyloxycarbonsäureamide als Herbizide verwendet werden können (vgl. z. B. DE-OS 29 14 003, DE-A-3 038 636 und DE-OS 30 04 326). So kann z. B. 4,5-Dichlor-1,3-thiazol-2-yl-oxyessigsäure-N,N-diethylamid zur selektiven Bekämpfung von Gräsern in dikotylen Kulturpflanzen, wie z. B. Baumwolle, eingesetzt werden ; gegen dikotyle Unkräuter ist die Verbindung jedoch nicht ausreichend wirksam. Trihalogenmethyl-1,2,4-thiadiazole mit herbizider Wirkung sind beschrieben in US-A-4 228 290.

Es wurden nun neue substituierte, fluorhaltige 3-Trihalogenmethyl-1,2,4-thiadiazol-5-yl-oxyessigsäureamide der Formel

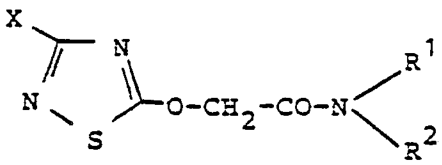

(I)

aufgefunden, in welcher

X für $-CFCl_2$, $-CF_2Cl$ oder $-CF_3$ steht und

$R^1$ und $R^2$ gleich oder verschieden sind und einzeln für Alkyl, Cyanoalkyl, Alkoxyalkyl, Alkenyl, Alkinyl, Alkoxy, jeweils mit bis zu 10 C-Atomen, Cycloalkyl oder Cycloalkenyl mit jeweils bis zu 12 C-Atomen, Aralkyl mit 1 oder 2 C-Atomen im Alkylteil und 6 oder 10 C-Atomen im Arylteil, welcher gegebenenfalls durch Halogen substituiert ist, oder für Aryl mit 6 oder 10 C-Atomen stehen, wobei der Arylrest durch 1 bis 3 Halogenatome, 1 bis 3 Alkyl- oder Halogenalkylgruppen mit jeweils 1 bis 4 C-Atomen, Nitro, Cyano oder Alkoxy mit 1 bis 4 C-Atomen substituiert sein kann, oder worin die Reste $R^1$ und $R^2$ zusammen mit dem Stickstoffatom, an das sie gebunden sind, einen gegebenenfalls durch 1 bis 3 Alkylgruppen mit jeweils 1 bis 5 C-Atomen substituierten, teilweise ungesättigten und/oder benzanellierten Monocyclus oder Bicyclus mit bis zu 15 C-Atomen bilden, oder worin die Reste $R^1$ und $R^2$ zusammen mit dem Stickstoffatom, an das sie gebunden sind, einen gegebenenfalls durch 1 bis 3 Alkylgruppen mit jeweils 1 bis 5 C-Atomen substituierten, gesättigten und gegebenenfalls ein weiteres Stickstoffatom, Sauerstoffatom oder Schwefelatom enthaltenden Monocyclus mit bis zu 6 C-Atomen bilden.

Man erhält die neuen Verbindungen der Formel (I), wenn man Hydroxyessigsäureamide der Formel

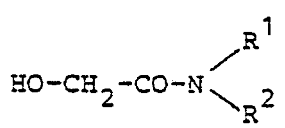

(II)

in welcher $R^1$ und $R^2$ die oben angegebene Bedeutung haben, mit einem fluorhaltigen 5-Chlor-3-trihalogenmethyl-1,2,4-thiadiazol der Formel

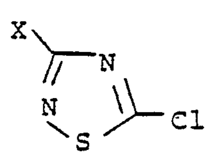

(III)

in welcher X die oben angegebene Bedeutung hat, in Gegenwart eines Säureakzeptors und gegebenenfalls unter Verwendung eines Verdünnungsmittels umsetzt.

Die neuen substituierten, fluorhaltigen 3-Trihalogenmethyl-1,2,4-thiadiazol-5-yl-oxyessigsäureamide der Formel (I) zeichnen sich durch starke herbizide Wirksamkeit aus.

Überraschenderweise sind die erfindungsgemäßen Wirkstoffe der Formel (I) außer gegen Gräser auch gegen dikotyle Unkräuter sehr wirksam. Die neuen Wirkstoffe haben somit wesentliche Vorteile gegenüber den vorbekannten Azolyloxycarbonsäureamiden (gemäß DE-OS 29 14 003 und 30 04 326), welche im wesentlichen nur gegen Gräser wirken.

Gegenstand der Erfindung sind vorzugsweise 3-Trihalogenmethyl-1,2,4-thiadiazol-5-yl-oxyessigsäureamide der Formel (I), in welcher.

$R^1$ für $C_1$-$C_5$-Alkyl, Cyanoethyl, $C_1$-$C_4$-Alkoxy-ethyl, Allyl, Propargyl, 1-Methyl-propargyl oder 1,1-Dimethyl-propargyl steht und

$R^2$ für $C_1$-$C_5$-Alkyl, $C_1$-$C_5$-Alkoxy, Cyanoethyl, $C_1$-$C_4$-Alkoxy-ethyl, Allyl, Propargyl, 1-Methyl-propargyl, 1,1-Dimethylpropargyl, Cyclopentyl, Cyclohexyl, 3,4,6-Trimethyl-cyclohexen-1-yl, Benzyl, Naphthyl

oder Phenyl, welches gegebenenfalls durch 1 bis 3 Reste (Methyl, Chlor, Fluor, Trifluormethyl, Methoxy, Methylthio, Trifluormethoxy und/oder Trifluormethylthio) substituiert ist, steht
oder worin die Reste $R^1$ und $R^2$ zusammen mit dem Stickstoffatom, an das sie gebunden sind, für Pyrrolidyl, Monoalkyl- oder Dialkyl-pyrrolidyl mit 1 bis 3 Kohlenstoffatomen je Alkylgruppe, Morpholinyl oder Dialkylmorpholinyl mit 1 bis 3 Kohlenstoffatomen je Alkylgruppe, Piperidyl, Monoalkyl-, Dialkyl- oder Trialkylpiperidyl mit 1 bis 3 Kohlenstoffatomen je Alkylgruppe, für Perhydroazepinyl (Hexamethylenimino-Rest), für den Heptamethylenimino-Rest, für 1,2,3,4-Tetrahydroindolyl, für Monoalkyl- oder Dialkyl-tetrahydroindolyl mit bis zu 3 Kohlenstoffatomen je Alkylgruppe, für Perhydroindolyl, Monoalkyl- oder Dialkylperhydroindolyl mit 1 bis 3 Kohlenstoffatomen je Alkylgruppe, für 1,2,3,4-Tetrahydrochinolyl oder 1,2,3,4-Tetrahydro-iso-chinolyl, Monoalkyl- oder Dialkyl-1,2,3,4-tetrahydrochinolyl oder -iso-chinolyl mit 1 bis 3 Kohlenstoffatomen je Alkylgruppe, für Perhydrochinolyl oder Perhydro-iso-chinolyl, Monoalkyl- oder Dialkyl-perhydrochinolyl oder -perhydroisochinolyl mit 1 bis 3 Kohlenstoffatomen je Alkylgruppe stehen.

Verwendet man 5-Chlor-3-fluordichlormethyl-1,2,4-thiadiazol und beispielsweise Hydroxyessigsäure-N-methylanilid als Ausgangsstoffe, so kann der Reaktionsablauf nach dem erfindungsgemäßen Verfahren durch folgendes Formelschema wiedergegeben werden :

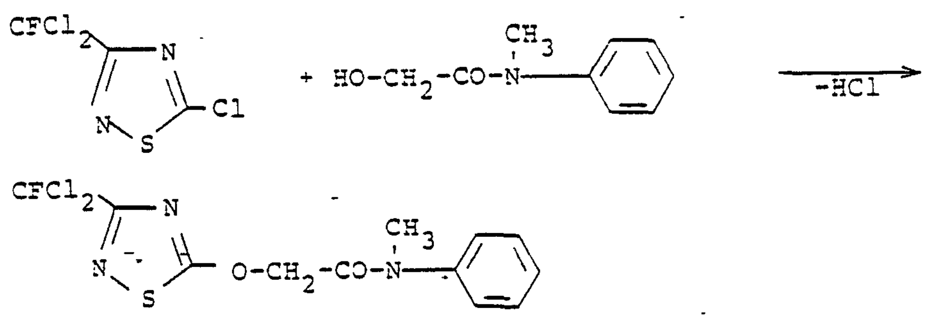


Die als Ausgangsstoffe zu verwendenden Hydroxyessigsäureamide sind durch Formel (II) definiert. In dieser Formel stehen $R^1$ und $R^2$ vorzugsweise bzw. insbesondere für diejenigen Reste, welche bereits im Rahmen der Substituentendefinitionen der Formel (I) vorzugsweise bzw. als insbesondere bevorzugt genannt sind.

Als Ausgangsstoffe der Formel (II) seien beispielsweise genannt :

Hydroxyessigsäure-dimethylamid, -diethylamid, -di-n-propylamid, -di-iso-propylamid, -N-methyl-N-iso-propylamid, -N-methyl-N-isobutyl-amid, -N-methyl-N-sek.-butylamid, -N-propyl-N-sek.-butyl-amid, -N-methyl-N-(2-cyanoethyl)-amid, -di-(2-methoxy-ethyl)-amid, -di-allyl-amid, -N-methyl-N-propargyl-amid, -N-methyl-N-(1-methyl-propargyl)-amid, -dipropargyl-amid, -N-methyl-N-cyclopentyl-amid, -N-methyl-N-cyclohexyl-amid, -N-methyl-N-(2-nitro-phenyl)-, -N-methyl-N-(3-nitro-phenyl)-, -N-methyl-N-(4-nitro-phenyl)-amid, -N-methyl-N-(2-chlor-phenyl)-, -N-methyl-N-(3-chlor-phenyl)-, -N-methyl-N-(4-chlor-phenyl)-amid, -N-methyl-N-(3-nitro-6-methyl-phenyl)-amid, -N-ethyl-anilid, -N-ethyl-N-(2-nitro-phenyl)-, -N-ethyl-(3-nitro-phenyl)-, -N-ethyl-N-(4-nitro-phenyl)-amid, -N-ethyl-N-(2-chlor-phenyl)-, -N-ethyl-N-(3-chlor-phenyl)-, -N-ethyl-N-)4-chlor-phenyl)-amid, -N-ethyl-N-(3-nitro-6-methyl-phenyl)-amid, -N-propyl-anilid, -N-propyl-N-(2-nitro-phenyl)-, N-propyl-N-(3-nitro-phenyl)-, -N-propyl-N-(4-nitro-phenyl)-amid, -N-propyl-N-(2-chlor-phenyl)-, -N-propyl-N-(3-chlor-phenyl)-, -N-propyl-N-(4-chlor-phenyl)-amid, -N-propyl-N-(2-methyl-phenyl)-, -N-propyl-N-(3-methyl-phenyl)-, -N-propyl-N-(4-methyl-phenyl)-amid, -N-propyl-N-(3-nitro-6-methyl-phenyl)-amid, -N-butyl-anilid, -N-methyl-N-naphth(1)ylamid, -N-methyl-N-naphth(2)ylamid, -N-ethyl-N-naphth(1)ylamid, -N-ethyl-N-naphth(2)ylamid, -N-methyl-N-benzylamid, -N-ethyl-N-benzylamid, -N-propyl-N-benzylamid, -N-butyl-N-benzylamid, -pyrrolidid, -1-methyl-pyrrolidid, -morpholid, -piperidid, -2-methyl-piperidid, -2-methyl-piperidid, -4-methyl-piperidid, -2,4-dimethyl-piperidid, -3,5-Dimethyl-piperidid, -3,5-Diethylpiperidid, -2,4,6-trimethyl-piperidid, -2-ethyl-piperidid, -4-ethyl-piperidid, -2,4-diethyl-piperidid, -2,4,6-triethyl-piperidid, -2-methyl-4-ethyl-piperidid, -2-ethyl-4-methyl-piperidid, -2-methyl-5-ethyl-piperidid, -2-ethyl-5-methyl-piperidid, -2-methyl-6-ethyl-piperidid, -1,2,3,4-tetrahydroindolid, -2-methyl-1,2,3,4-tetrahydroindolid, -perhydroindolid, -2-methyl-perhydroindolid, 2,2-dimethyl-perhydroindolid, -2-methyl-1,2,3,4-tetrahydrochinolid, -perhydrochinolid, -2-methyl-perhydrochinolid, -1,2,3,4-tetrahydro-isochinolid und -perhydroisochinolid ; ferner -N-methyl-N-(2-methylthio-phenyl)-, -N-methyl-N-(3-methylthio-phenyl)- und -N-methyl-N-(4-methylthio-phenyl)-amid, -N-methyl-N-(2-fluorphenyl)-, -N-methyl-N-(3-fluorphenyl)- und -N-methyl-N-(4-fluorphenyl)-amid ; -N-methyl-N-(2-trifluormethylphenyl)-, -N-methyl-N-(3-trifluormethylphenyl)- und -N-methyl-N-(4-trifluormethylphenyl)-amid ; -N-methyl-N-(2-trifuormethoxyphenyl)-, -N-methyl-N-(3-trifluormethoxyphenyl)- und -N-methyl-N-(4-trifluormethoxyphenyl)-amid ; -N-methyl-N-(2-trifluorethylphenyl)-amid.

Die Hydroxy-carbonsäureamide der Formel (II) sind teilweise bekannt (vgl. US-PS 3 399 998 ; DE-OS'en 2 201 432 und 2 647 481).' Sie können, wie im nachstehenden Schema skizziert, ausgehend von

Chloracetylchlorid hergestellt werden :

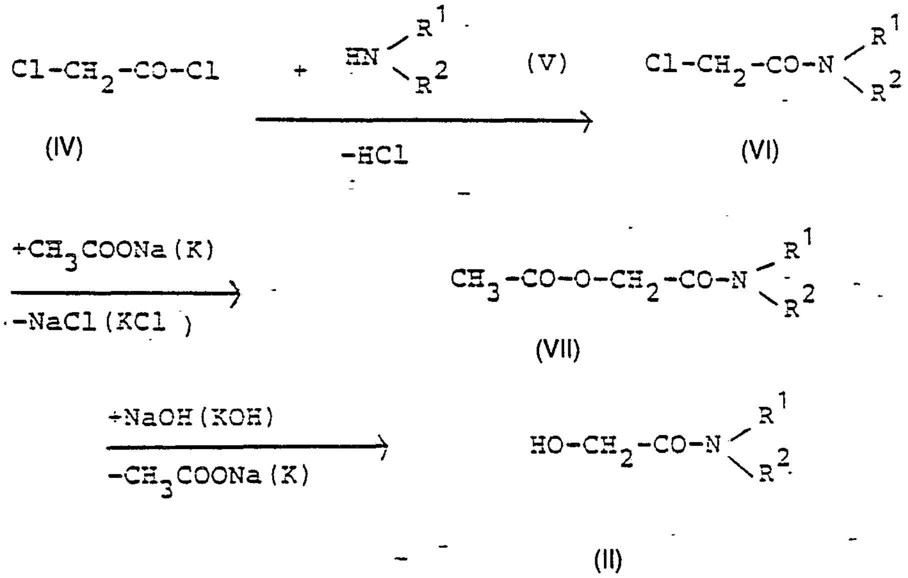


Hierzu wird zunächst das literaturbekannte Chloracetylchlorid der Formel (IV) mit Aminen der Formel (V) — wobei $R^1$ und $R^2$ die oben angegebene Bedeutung haben — gegebenenfalls in Gegenwart eines Säurebindemittels wie z. B. Triethylamin und gegebenenfalls unter Verwendung eines inerten Verdünnungsmittels wie z. B. 1,2-Dichlorethan, bei Temperaturen zwischen — 20 und 100 °C, vorzugsweise zwischen — 10 und 50 °C in die entsprechenden Chloressigsäureamide der Formel (VI) überführt. Die Aufarbeitung dieser Produkte erfolgt nach üblichen Methoden durch Waschen mit Wasser, Trocknen der organischen Phase und Abdestillieren des Lösungsmittels.

Die Verbindungen der Formel (VI) werden mit Natrium- oder Kalium-acetat, gegebenenfalls unter Verwendung eines Verdünnungsmittels wie z. B. Essigsäure oder Dimethylsulfoxid, bei Temperaturen zwischen 20 und 150 °C, vorzugsweise zwischen 50 und 120 °C, zu den entsprechenden Acetoxyessigsäureamiden der Formel (VII) umgesetzt. Soweit die Produkte hierbei kristallin anfallen, werden sie durch Absaugen isoliert. Andernfalls erfolgt die Aufarbeitung nach üblichen Methoden, beispielsweise durch Abdestillieren des Lösungsmittels im Vakuum, Aufnehmen des Rückstandes in Methylenchlorid, Waschen mit Wasser und Abdestillieren des Lösungsmittels.

Durch Umsetzung mit wäßrig-alkoholischer Natronlauge oder Kalilauge bei Temperaturen zwischen 0 und 100 °C, vorzugsweise zwischen 10 und 50 °C, können die Verbindungen der Formel (VII) zu den Verbindungen der Formel (II) entacyliert werden. Zur Isolierung der Produkte werden die Lösungsmittel im Vakuum abdestilliert, der Rückstand mit einem organischen Lösungsmittel wie z. B. Methylenchlorid oder Essigester extrahiert, die Lösung getrocknet und das Lösungsmittel abdestilliert.

Die als Ausgangsstoffe zu verwendenden fluorhaltigen 5-Chlor-3-trihalogenmethyl-1,2,4-thiadiazole sind durch die Formel (III) definiert. Die Verbindung (III) mit $X = CF_3$ [5-Chlor-3-trifluormethyl-1,2,4-thiadiazol (IIIc)] und ein Verfahren zu ihrer Herstellung sind bekannt (vgl. J. Org. Chem. *27*, S. 2589-2592 (1962)). Diese Verbindung kann hergestellt werden durch Umsetzung von 5-Chlor-3-trichlormethyl-1,2,4-thiadiazol (IV) (vgl. auch US-PS 3 260 588) mit Antimontrifluorid.

Die Verbindungen der Formel (III) mit $X = CFCl_2$ und mit $X = CF_2Cl$ sind noch nicht beschrieben worden. Sie können nach einem neuen, erfinderischen Verfahren hergestellt werden, indem man das vorbekannte 5-Chlor-3-trichlormethyl-1,2,4-thiadiazol der Formel

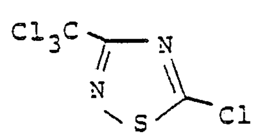

(IV)

in einem Druckgefäß mit überschüssigem, wasserfreiem Fluorwasserstoff bei Temperaturen zwischen 100 und 180 °C und bei Drucken bis etwa 50 bar umsetzt und die Produkte durch fraktionierte Destillation isoliert.

Hierbei setzt man im allgemeinen auf 1 Mol der Verbindung (IV) 8-30 Mol, vorzugsweise 15-20 Mol, Fluorwasserstoff ein.

Bei der stattfindenden Cl/F-Austauschreaktion wird im Temperaturbereich von 100-140 °C vorzugsweise die Verbindung (III) mit $X = CFCl_2$ [5-Chlor-3-fluordichlormethyl-1,2,4-thiadiazol (IIIa)] gebildet. Im Temperaturbereich von 140-180 °C bildet sich bevorzugt die Verbindung (III) mit $X = CF_2Cl$ [5-

Chlor-3-difluorchlormethyl-1,2,4-thiadiazol (IIIb)].

Daneben entsteht bei dieser Umsetzung im Temperaturbereich von etwa 160-180 °C in geringer Menge von etwa 2-4 % auch die bekannte Verbindung (IIIc), die ebenfalls durch fraktionierte Destillation abtrennbar ist.

Weiterhin wurde gefunden, daß man die Verbindung (IIIc) in guten Ausbeuten erhält, indem man die Verbindung (IIIa) oder (IIIb) oder ein Gemisch dieser Verbindungen mit Antimontrifluorid in Gegenwart katalytischer Mengen von Antimonpentachlorid umsetzt. Man gibt die Reaktionspartner bei Raumtemperatur zusammen, erhitzt das Reaktionsgemisch bis auf eine Endtemperatur von etwa 160 °C und beobachtet schließlich einen Temperaturabfall auf etwa 130 °C entsprechend dem Siedepunkt des gebildeten Produktes (IIIc). Bei der Durchführung dieser Nachfluorierung setzt man pro Mol der Verbindung (IIIa) bzw. (IIIb) das Antimontrifluorid ($SbF_3$) in einer Menge von 10-15 Mol-% Überschuß über die jeweils theoretisch berechnete, stöchiometrische Menge ein. Der Katalysator $SbCl_5$ wird in Mengen von 1-6 Gew.-%, vorzugsweise von 3 bis 5 Gew.-%, jeweils bezogen auf die Einsatzmenge an $SbF_3$, eingesetzt.

Die zur Herstellung der Ausgangsprodukte (IIIa), (IIIb) und (IIIc) beschriebenen Umsetzungen können zusammenfassend durch das folgende Formelschema wiedergegeben werden :

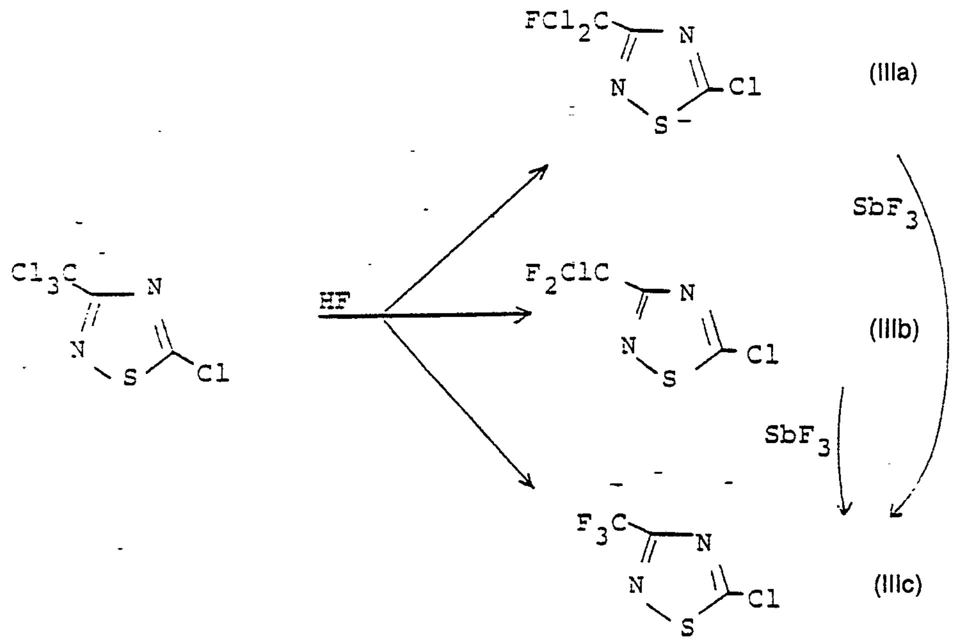


(IIIa)

(IIIb)

(IIIc)

Weitere Einzelheiten hierzu sind den Herstellungsbeispielen zu entnehmen.

Das Verfahren zur Herstellung der neuen Verbindungen der Formel (I) wird vorzugsweise unter Verwendung geeigneter Lösungs- oder Verdünnungsmittel durchgeführt. Als solche kommen praktisch alle inerten organischen Solventien in Frage. Hierzu gehören insbesondere Alkohole, wie Methanol, Ethanol, n- und iso-Propanol, n-, iso-, sek.- und tert.-Butanol, Ether wie Diethylether, Dibutylether, Tetrahydrofuran, Diglyme und Dioxan, Ketone wie Aceton, Methylethylketon, Methylisopropylketon und Methylisobutylketon, Nitrile wie Acetonitril und Propionsäurenitril, sowie die hochpolaren Lösungsmittel Dimethylformamid, Dimethylsulfoxid, Sulfolan und Hexamethylphosphorsäuretriamid.

Als Säureakzeptoren können praktisch alle üblicherweise verwendbaren Säurebindemittel eingesetzt werden : hierzu gehören insbesondere Alkali- und Erdalkalihydroxide bzw. -oxide wie Natrium-, Kaliumhydroxid und insbesondere Lithiumhydroxid sowie Calciumoxid oder Calcium-hydroxid, Alkali- und Erdalkali-carbonate wie Natrium-, Kalium- und Calciumcarbonat, Alkalialkoholate wie Natriummethylat, -ethylat und -tert-butylat, Kaliummethylat, -ethylat und -tert.-butylat, ferner aliphatische, aromatische oder heterocyclische Amine wie Triethylamin, Dimethylanilin, Dimethylbenzylamin, Pyridin, Diazabicyclooctan und Diazabicycloundecen.

Die Reaktionstemperatur kann bei dem erfindungsgemäßen Verfahren zur Herstellung der Wirkstoffe (I) innerhalb eines größeren Bereichs variiert werden. Im allgemeinen arbeitet man zwischen — 50 und + 150 °C, vorzugsweise bei — 20 bis + 100 °C. Das erfindungsgemäße Verfahren wird im allgemeinen bei Normaldruck durchgeführt.

Bei der Durchführung des erfindungsgemäßen Verfahrens setzt man auf 1 Mol 5-Chlor-3-trihalogenmethyl-1,2,4-thiadiazol der Formel (III) 1,0 bis 1,5 Mol Hydroxyessigsäureamid der Formel (II) ein. Die Umsetzung wird im allgemeinen in einem geeigneten Verdünnungsmittel durchgeführt und das Reaktionsgemisch wird bei der erforderlichen Temperatur mehrere Stunden gerührt.

Die Isolierung der Produkte erfolgt nach üblichen Methoden : Man destilliert gegebenenfalls einen Teil des Verdünnungsmittels unter vermindertem Druck ab und gießt den Rest der Reaktionsmischung in

Wasser. Soweit die Produkte hierbei kristallin anfallen, werden sie durch Absaugen isoliert. Andernfalls werden die organischen Produkte mit einem mit Wasser nicht mischbaren Lösungsmittel wie z. B. Toluol oder Methylenchlorid extrahiert ; nach Waschen und Trocknen wird dann von der organischen Phase das Lösungsmittel im Vakuum abdestilliert. Die zurückleibenden Produkte werden durch ihren Schmelzpunkt bzw. ihren Brechungsindex charakterisiert.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden :

Dikotyle Unkräuter der Gattungen : Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen : Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen : Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen : Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen neben einer sehr guten Wirkung gegen monokotyle Umkräuter auch eine gute herbizide Wirkung bei dikotylen Umkräutern. Ein selektiver Einsatz der erfindungsgemäßen Wirkstoffe ist in verschiedenen Kulturen möglich, vor allem in dikotylen Kulturpflanzen wie z. B. Baumwolle, sowie in Reis und Getreide.

Die Wirkstoffe können in die üblichen Formulierungen übergeführt werden, wie Lösungen, Emulsionen, Spritzpulver, Suspension, Pulver, Stäubemittel, Pasten, lösliche Pulver, Granulate, Suspensions-Emulsions-Konzentrate, wirkstoff-imprägnierte Natur- und synthetische Stoffe, Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage :

z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silicate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyethylen-Fettsäure-Ester, Polyoxyethylen-Fettalkohol-Ether, z. B. Alkylaryl-polyglykol-ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen oder Stäuben.

Die erfindungsgemäßen Wirkstoffe können sowohl vor als auch nach dem Auflaufen der Pflanzen appliziert werden.

Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effektes ab. Im allgemeinen liegen die Aufwandmengen zwischen 0,01 und 10 kg Wirkstoff pro ha, vorzugsweise zwischen 0,1 und 5 kg/ha.

Die erfindungsgemäßen Wirkstoffe können in niedriger Dosierung auch als Wachstumsregulatoren verwendet werden.

Die nachfolgenden Beispiele dienen zur weiteren Erläuterung der Erfindung.

## Herstellungsbeispiele

### Beispiel 1

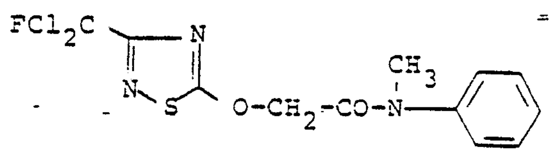

(1)

13 g (0,05 Mol) 5-Chlor-3-fluordichlormethyl-1,2,4-thiadiazol werden zu einer auf — 10 °C gekühlten Mischung aus 8,2 g (0,05 Mol) Hydroxyessigsäure-N-methylanilid, 3,1 g (0,05 Mol) Kaliumhydroxid-Pulver und 80 ml iso-Propanol gegeben und das Reaktionsgemisch wird 4 Stunden bei — 10 °C gerührt. Dann wird mit Wasser verdünnt und abgesaugt. Man erhält 13 g (74 % der Theorie) (3-Fluor-dichlormethyl-1,2,4-thiadiazol-5-yl)-oxyessigsäure-N-methylanilid in Form weißer Kristalle vom Schmelzpunkt 67 °C.

### Beispiel 2

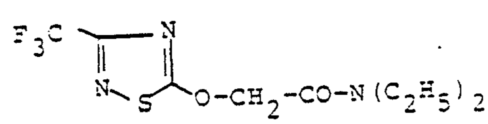

(2)

5,6 g (0,03 Mol) 5-Chlor-3-trifluormethyl-1,2,4-thiadiazol werden zu einer auf — 10 °C gekühlten Mischung aus 4 g (0,03 Mol) Hydroxyessigsäure-diethylamid, 1,9 g (0,03 Mol) Kaliumhydroxid-Pulver und 80 ml iso-Propanol gegeben. Das Reaktionsgemisch wird 10 Stunden bei — 10 °C gerührt, in Wasser gegossen und abgesaugt. Man erhält 4 g (47 % der Theorie) (3-Trifluormethyl-1,2,4-thiadiazol-5-yl)-oxyessigsäure-diethylamid in Form weißer Kristalle vom Schmelzpunkt 60 °C.

Analog Beispielen 1 und 2 können die in der nachfolgenden Tabelle 1 aufgeführten Verbindungen der Formel (I) hergestellt werden :

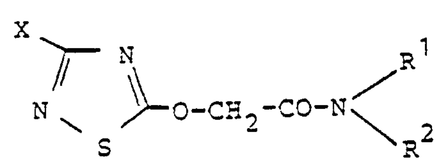

(I)

Tabelle 1

| Verbindung Nr. | -X | 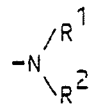 | Schmelzpunkt °C |
|---|---|---|---|
| 3 | $-CF_2Cl$ | 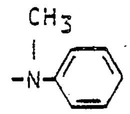 | 75 |
| 4 | $-CF_3$ | 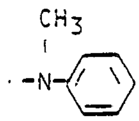 | 91 |
| 5 | $-CF_3$ | 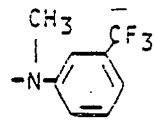 | 103 |
| 6 | $-CF_3$ | 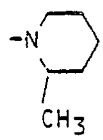 | 76 |
| 7 | $-CF_3$ | 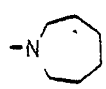 | 76 |
| 8 | $-CF_3$ | 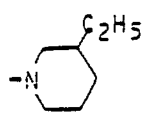 | 57 |
| 9 | $-CF_3$ | 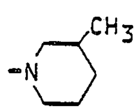 | 70 |
| 10 | $-CF_3$ | 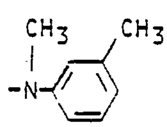 | 41 |
| 11 | $-CFCl_2$ | $-N(C_2H_5)_2$ | 48 |
| 12 | $-CFCl_2$ | 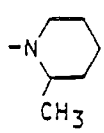 | $n_D^{20}$: 1,5251 |
| 13 | $-CFCl_2$ | 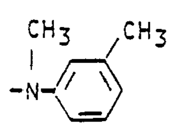 | $n_D^{20}$: 1,5458 |

Tabelle 1 (Fortsetzung)

| Verbindung Nr. | -X· | 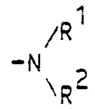 | Schmelzpunkt °C oder $n_D^{20}$ (Brechungsindex) |
|---|---|---|---|
| 14 | $-CFCl_2$ | 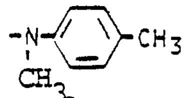 | 112 |
| 15 | $-CFCl_2$ | 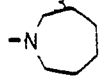 | 88 |
| 16 | $-CFCl_2$ | 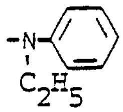 | 48 |
| 17 | $-CFCl_2$ | 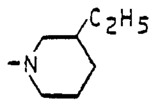 | 64 |
| 18 | $-CFCl_2$ | 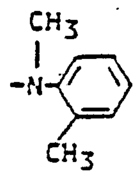 | 50 |
| 19 | $-CFCl_2$ | 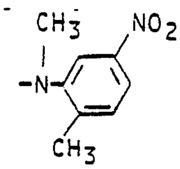 | 90 |
| 20 | $-CF_3$ | 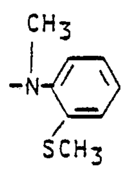 | |
| 21 | $-CFCl_2$ | 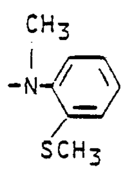 | |
| 22 | $-CF_2Cl$ | 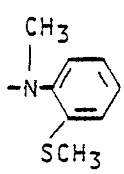 | |
| 23 | $-CF_3$ | 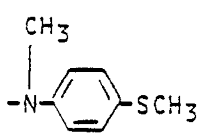 | |

Tabelle 1 (Fortsetzung)

| Verbindung Nr. | -X | 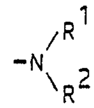 | Schmelzpunkt °C |
|---|---|---|---|
| 24 | $-CFCl_2$ | 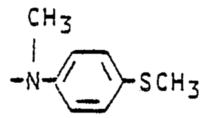 | |
| 25 | $-CF_2Cl$ | 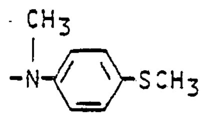 | |
| 26 | $-CF_3$ | 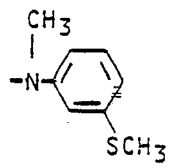 | |
| 27 | $-CFCl_2$ | 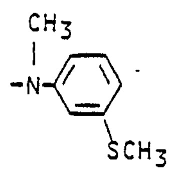 | |
| 28 | $-CF_2Cl$ | 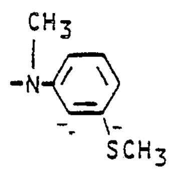 | |
| 29 | $-CF_3$ | 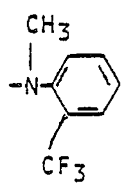 | |
| 30 | $-CFCl_2$ | 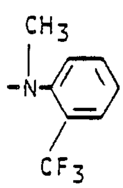 | |
| 31 | $-CF_2Cl$ | 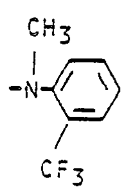 | |
| 32 | $-CF_3$ | 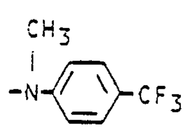 | |

Tabelle 1 (Fortsetzung)

| Verbindung Nr. | -X | 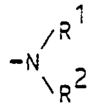 |
|---|---|---|
| 33 | $-CFCl_2$ | 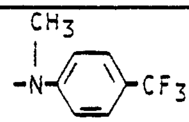 |
| 34 | $-CF_2Cl$ | 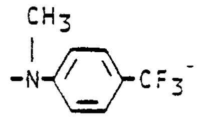 |
| 35 | $-CF_3$ | 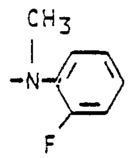 |
| 36 | $-CFCl_2$ | 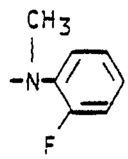 |
| 37 | $-CF_2Cl$ | 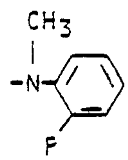 |
| 38 | $-CF_3$ | 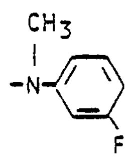 |
| 39 | $-CFCl_2$ | 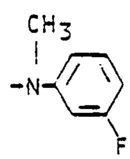 |
| 40 | $-CF_2Cl$ | 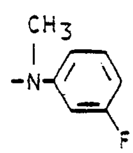 |
| 41 | $-CF_3$ | 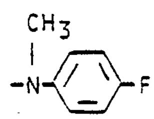 |
| 42 | $-CFCl_2$ | 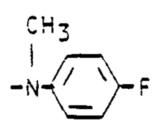 |

Tabelle 1 (Fortsetzung)

| Verbindung Nr. | -X | 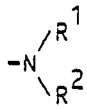 |
|---|---|---|
| 43 | $-CF_2Cl$ | 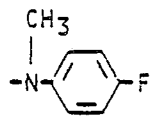 |
| 44 | $-CF_3$ | 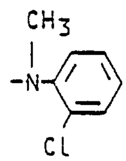 |
| 45 | $-CFCl_2$ | 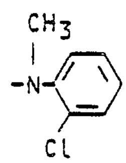 |
| 46 | $-CF_2Cl$ | 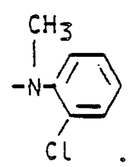 |
| 47 | $-CF_3$ | 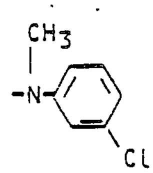 |
| 48 | $-CFCl_2$ | 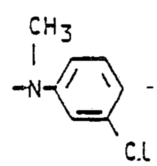 |
| 49 | $-CF_2Cl$ | 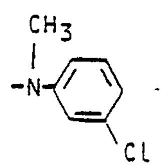 |
| 50 | $-CF_3$ | 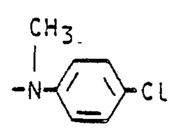 |
| 51 | $-CFCl_2$ | 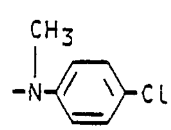 |
| 52 | $-CF_2Cl$ | 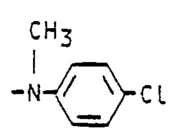 |

Tabelle 1 (Fortsetzung)

| Verbindung Nr. | -X | 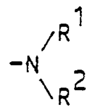 |
|---|---|---|
| 53 | $-CF_3$ | 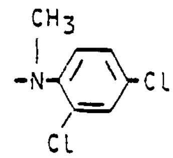 |
| 54 | $-CFCl_2$ | 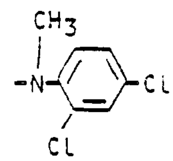 |
| 55 | $-CF_2Cl$ | 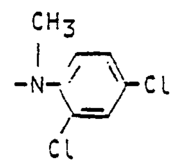 |
| 56 | $-CF_3$ | 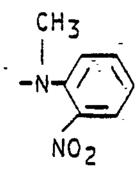 |
| 57 | $-CFCl_2$ | 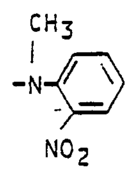 |
| 58 | $-CF_2Cl$ | 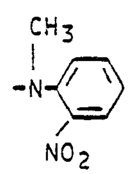 |
| 59 | $-CF_3$ | 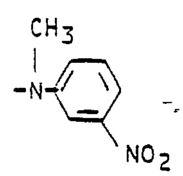 |
| 60 | $-CFCl_2$ | 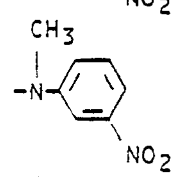 |
| 61 | $-CF_2Cl$ | 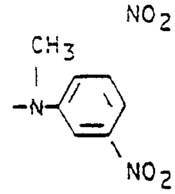 |

Tabelle 1 (Fortsetzung)

| Verbindung Nr. | -X | 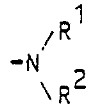 |
|---|---|---|
| 62 | $-CF_3$ | 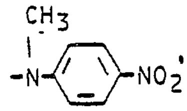 |
| 63 | $-CFCl_2$ | 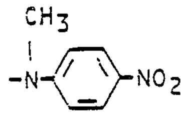 |
| 64 | $-CF_2Cl$ | 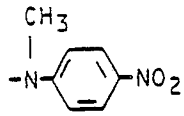 |
| 65 | $-CF_3$ | 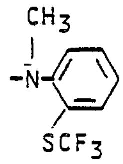 |
| 66 | $-CFCl_2$ | 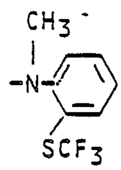 |
| 67 | $-CF_2Cl$ | 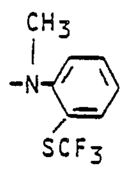 |
| 68 | $-CF_3$ | 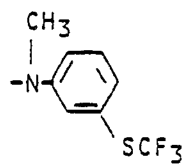 |
| 69 | $-CFCl_2$ | 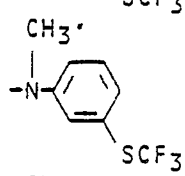 |
| 70 | $-CF_2Cl$ | 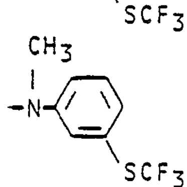 |
| 71 | $-CF_3$ | 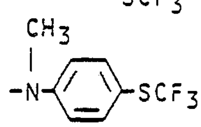 |
| 72 | $-CFCl_2$ | 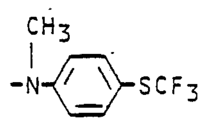 |

Tabelle 1 (Fortsetzung)

| Verbindung Nr. | -X | 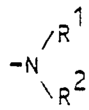 |
|---|---|---|
| 73 | $-CF_2Cl$ | 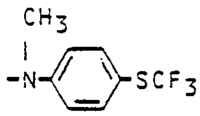 |
| 74 | $-CF_3$ | 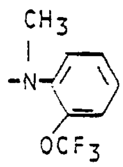 |
| 75 | $-CFCl_2$ | 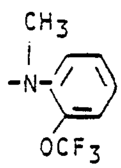 |
| 76 | $-CF_2Cl$ | 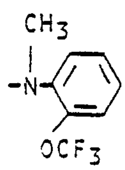 |
| 77 | $-CF_3$ | 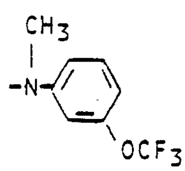 |
| 78 | $-CFCl_2$ | 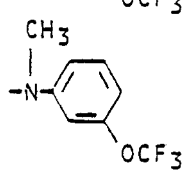 |
| 79 | $-CF_2Cl$ | 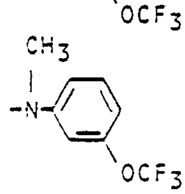 |
| 80 | $-CF_3$ | 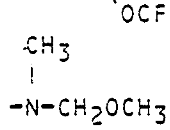 |
| 81 | $-CFCl_2$ | 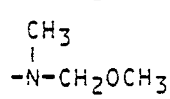 |
| 82 | $-CF_2Cl$ | 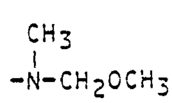 |
| 83 | $-CF_3$ | 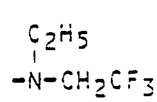 |

Tabelle 1 (Fortsetzung)

| Verbindung Nr. | -X | $-N(R^1)(R^2)$ | Schmelzpunkt °C |
|---|---|---|---|
| 84 | $-CFCl_2$ | 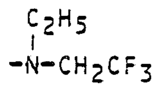 | |
| 85 | $-CF_2Cl$ | 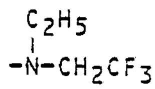 | |
| 86 | $-CF_3$ | 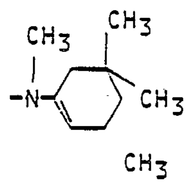 | |
| 87 | $-CFCl_2$ | 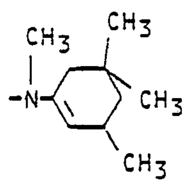 | |
| 88 | $-CF_2Cl$ | 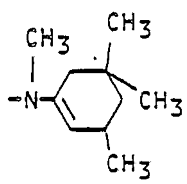 | |
| 89 | $-CF_3$ | 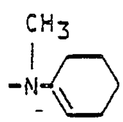 | 50 |
| 90 | $-CFCl_2$ | 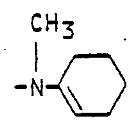 | |
| 91 | $-CF_2Cl$ | 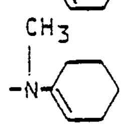 | |
| 92 | $-CF_3$ | $-N(CH_3)_2$ | 60 |
| 93 | $-CF_3$ | 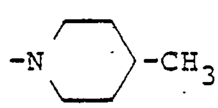 | 43 |
| 94 | $-CF_3$ | 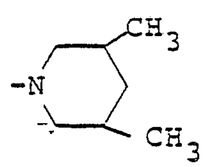 | 52 |

Tabelle 1 (Fortsetzung)

| Verbindung Nr. | X- | 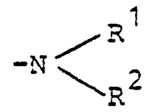 | Schmelzpunkt °C (Brechungsindex) |
|---|---|---|---|
| 95 | $-CF_3$ | 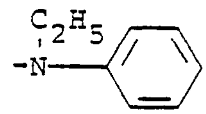 | ($n_D^{20}$: 1.5094) |
| 96 | $-CF_3$ | 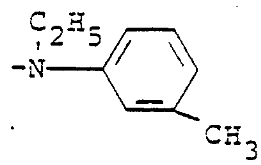 | 50 |
| 97 | $-CF_3$ | 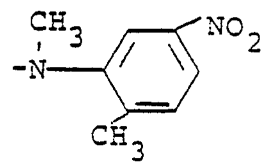 | 115 |
| 98 | $-CF_3$ | 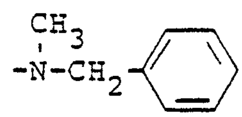 | 48 |
| 99 | $-CF_2Cl$ | $-N(CH_3)_2$ | 55 |
| 100 | $-CF_2Cl$ | 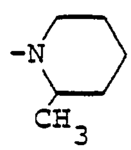 | ($n_D^{20}$ : 1.4979) |
| 101 | $-CF_2Cl$ | $-N(C_2H_5)_2$ | 46 |
| 102 | $-CF_2Cl$ | $-N(CH_2-CH=CH_2)_2$ | ($n_D^{20}$ : 1.5029) |
| 103 | $-CF_2Cl$ | 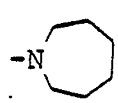 | 80 |
| 104 | $-CF_2Cl$ | 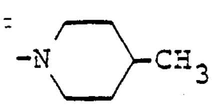 | 54 |
| 105 | $-CF_2Cl$ | 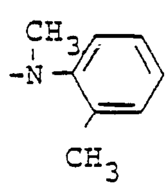 | 48 |

Herstellung der Ausgangsstoffe der Formel (III)

A) Herstellung von 3-Fluordichlormethyl-5-chlor-1,2,4-thiadiazol (IIIa) und 3-Difluorchlormethyl-5-chlor-1,2,4-thiadiazol (IIIb)

In einem Autoklaven werden bei 0 °C 4 520 g 3-Trichlormethyl-5-chlor-1,2,4-thiadiazol (IV) und 3 800 ml Fluorwasserstoff (abs.) vorgelegt.

Nach Verschließen des Autoklaven wird ein Stickstoff-Schutzdruck von 2 bar erzeugt und unter Rühren wird bis auf eine Innentemperatur von 140 °C erhitzt. Durch den im Reaktionsgemisch stattfindenden Cl/F-Austausch wird Chlorwasserstoff frei und es erfolgt ein entsprechender Druckanstieg. Über ein Regulierventil wird durch Entspannen ein Druck von ca. 28 bar eingehalten. Die Reaktion ist nach 5 Stunden beendet. Unter Heraufsetzen des Entspannungsdruckes auf 38 bar wird dann auf 160 °C geheizt und man läßt 6 Stunden bei dieser Temperatur weiterreagieren. Anschließend läßt man abkühlen, entspannt und arbeitet den Autoklaveninhalt durch Destillation auf.

Nach einem Vorlauf von unverbrauchtem Fluorwasserstoff werden folgende Fraktionen erhalten :

Fraktion 1

77 g, bis zu einem Siedepunkt von 54 °C/14 mbar ; enthält ca. 40 % 3-Trifluormethyl-5-chlor-1,2,4-thiadiazol (IIIc). Durch Redestillation dieser Fraktion kann (IIIc) isoliert werden ; Siedepunkt : 127 °C ; $n_D^{20}$ : 1,436 0.

Fraktion 2

2 280 g 3-Difluorchlormethyl-5-chlor-1,2,4-thiadiazol (IIIb) ; Siedepunkt : 55-56 °C/14 mbar ; $n_D^{20}$ : 1,482 2.

Fraktion 3

83 g Zwischenlauf

Fraktion 4

1 070 g 3-Fluordichlormethyl-5-chlor-1,2,4-thiadiazol (IIIa) ; Siedepunkt : 84 °C/16 mbar ; $n_D^{20}$ : 1.527 2.

B) Herstellung von 3-Trifluormethyl-5-chlor-1,2,4-thiadiazol (IIIc) :

In einem Rührkolben werden 221,5 g 3-Fluordichlormethyl-5-chlor-1,2,4-thiadiazol (IIIa) und 144 g Antimontrifluorid ($SbF_3$) vorgelegt und bis auf 155 °C geheizt. Dann kühlt man ab bis auf 50 °C, gibt 3 ml Antimonpentachlorid ($SbCl_5$) zu und erwärmt wieder langsam. Bei etwa 100 °C setzt eine deutlich exotherme Reaktion ein und der Ansatz verflüssigt sich. Man läßt noch 1 Stunde bei 135-137 °C Innentemperatur nachrühren und arbeitet dann durch Destillation auf. Das Destillat wird mit verdünnter Salzsäure gewaschen und getrocknet ; es kann gewünschtenfalls redestilliert werden.

Ausbeute : 174 g (92,3 % der Theorie) 3-Trifluormethyl-5-chlor-1,2,4-thiadiazol (IIIc) ; $n_D^{20}$ : 1,437 0. Eine erneute Destillation ergibt einen Siedepunkt für (IIIc) von 126-128 °C ; $n_D^{20}$ : 1,436 5.

Beispiel A

Pre-emergence-Test

Lösungsmittel : 5 Gewichtsteile Aceton
Emulgator : 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

In diesem Test zeigen z. B. die folgenden Verbindungen gemäß Herstellungsbeispielen eine ausgezeichnete Wirksamkeit : (1), (2).

Beispiel

Wuchshemmung bei Gerste

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

In diesem Test zeigen z. B. die folgenden Verbindungen gemäß Herstellungsbeispielen eine hervorragende Wirksamkeit : (2)

Beispiel

Wuchshemmung bei Sojabohnen

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

In diesem Test zeigen z. B. die folgenden Verbindungen gemäß Herstellungsbeispielen eine hervorragende Wirksamkeit : (2)

**Patentansprüche**

1. Substituierte 3-Trihalogenmethyl-1,2,4-thiadiazol-5-yl-oxyessigsäureamide der Formel

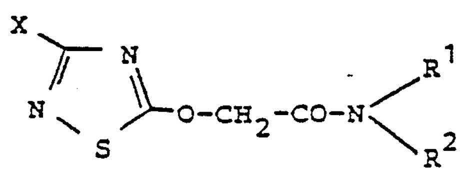

(I)

in welcher

X für $-CFCl_2$, $-CF_2Cl$ oder $-CF_3$ steht und

$R^1$ und $R^2$ gleich oder verschieden sind und einzeln für Alkyl, Cyanoalkyl, Alkoxyalkyl, Alkenyl, Alkinyl, Alkoxy, jeweils mit bis zu 10 C-Atomen, Cycloalkyl oder Cycloalkenyl mit jeweils bis zu 12 C-Atomen, Aralkyl mit 1 oder 2 C-Atomen im Alkylteil und 6 oder 10 C-Atomen im Arylteil, welcher gegebenenfalls durch Halogen substituiert ist, oder für Aryl mit 6 oder 10 C-Atomen stehen, wobei der Arylrest durch 1 bis 3 Halogenatome, 1 bis 3 Alkyl- oder Halogenalkylgruppen mit jeweils 1 bis 4 C-Atomen, Nitro, Cyano oder Alkoxy mit 1 bis 4 C-Atomen substituiert sein kann, oder worin die Reste $R^1$ und $R^2$ zusammen mit dem Stickstoffatom, an das sie gebunden sind, einen gegebenenfalls durch 1 bis 3 Alkylgruppen mit jeweils 1 bis 5 C-Atomen substituierten, teilweise ungesättigten und/oder benzanellierten Monocyclus oder Bicyclus mit bis zu 15 C-Atomen bilden, oder worin die Reste $R^1$ und $R^2$ zusammen mit dem Stickstoffatom, an das sie gebunden sind, einen gegebenenfalls durch 1 bis 3 Alkylgruppen mit jeweils 1 bis 5 C-Atomen substituierten, gesättigten und gegebenenfalls ein weiteres Stickstoffatom, Sauerstoffatom oder Schwefelatom enthaltenden Monocyclus mit bis zu 6 C-Atomen bilden.

2. Verbindungen der Formel (I) gemäß Anspruch 1, dadurch gekennzeichnet, daß

$R^1$ für $C_1$-$C_5$-Alkyl, Cyanoethyl, $C_1$-$C_4$-Alkoxy-ethyl, Allyl, Propargyl, 1-Methyl-propargyl oder 1,1-Dimethyl-propargyl steht und

$R^2$ für $C_1$-$C_5$-Alkyl, $C_1$-$C_5$-Alkoxy, Cyanoethyl, $C_1$-$C_4$-Alkoxy-ethyl, Allyl, Propargyl, 1-Methyl-propargyl, 1,1-Dimethylpropargyl, Cyclopentyl, Cyclohexyl, 3,4,6-Trimethyl-cyclohexen-1-yl, Benzyl, Naphthyl oder Phenyl, welches gegebenenfalls durch 1 bis 3 Reste (Methyl, Chlor, Fluor, Trifluormethyl, Methoxy, Methylthio, Trifluormethoxy und/oder Trifluormethylthio) substituiert ist, steht

oder worin die Reste $R^1$ und $R^2$ zusammen mit dem Stickstoffatom, an das sie gebunden sind, für Pyrrolidyl, Monoalkyl- oder Dialkyl-pyrrolidyl mit 1 bis 3 Kohlenstoffatomen je Alkylgruppe, Morpholinyl oder Dialkylmorpholinyl mit 1 bis 3 Kohlenstoffatomen je Alkylgruppe, Piperidyl, Monoalkyl-, Dialkyl- oder Trialkylpiperidyl mit 1 bis 3 Kohlenstoffatomen je Alkylgruppe, für Perhydroazepinyl (Hexamethylenimino-Rest), für den Heptamethylenimino-Rest, für 1,2,3,4-Tetrahydroindolyl, für Monoalkyl- oder Dialkyl-tetrahydroindolyl mit bis zu 3 Kohlenstoffatomen je Alkylgruppe, für Perhydroindolyl, Monoalkyl- oder Dialkylperhydroindolyl mit 1 bis 3 Kohlenstoffatomen je Alkylgruppe, für 1,2,3,4-Tetrahydrochinolyl oder 1,2,3,4-Tetrahydro-iso-chinolyl, Monoalkyl- oder Dialkyl-1,2,3,4-tetrahydrochinolyl oder -iso-chinolyl mit 1 bis 3 Kohlenstoffatomen je Alkylgruppe, für Perhydrochinolyl oder Perhydro-iso-chinolyl, Monoalkyl- oder Dialkyl-perhydrochinolyl oder -perhydroisochinolyl mit 1 bis 3 Kohlenstoffatomen je Alkylgruppe stehen.

3. (3-Fluordichlormethyl-1,2,4-thiadiazol-5-yl)-oxyessigsäure-N-methylanilid der Formel

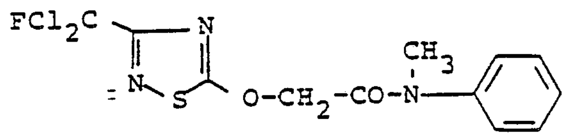

gemäß Anspruch 1.

4. (3-Trifluormethyl-1,2,4-thiadiazol-5-yl-oxyessigsäure-diethylamid der Formel

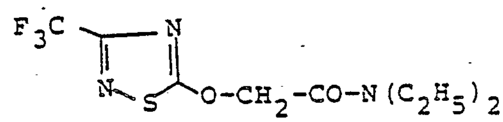

gemäß Anspruch 1.

5. (3-Trifluormethyl-1,2,4-thiadiazol-5-yl)-oxyessigsäure-N-ethyl-N-(2.2.2-trifluorethyl)-amid, (3-Fluordichlormethyl-1,2,4-thiadiazol-5-yl)-oxyessigsäure-N-ethyl-N-(2.2.2-trifluorethyl)-amid und (3-Difluorchlormethyl-1,2,4-thiadiazol-5-yl)-oxyessigsäure-N-ethyl-N-(2.2.2-trifluorethyl)-amid.

6. Verfahren zur Herstellung von substituierten 3-Trihalogenmethyl-1,2,4-thiadiazol-5-yl-oxyessigsäureamiden der Formel (I) gemäß Anspruch 1, dadurch gekennzeichnet, daß man Hydroxyessigsäureamide der Formel

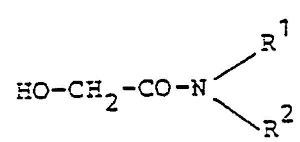

(II)

in welcher $R^1$ und $R^2$ die oben angegebene Bedeutung haben, mit einem fluorhaltigen 5-Chlor-3-trihalogenmethyl-1,2,4-thiadiazol der Formel

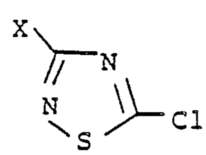

(III)

in welcher X die oben angegebene Bedeutung hat, in Gegenwart eines Säureakzeptors und gegebenenfalls unter Verwendung eines Verdünnungsmittels umsetzt.

7. Herbizide Mittel, gekennzeichnet durch einen Gehalt an substituierten 3-Trihalogenmethyl-1,2,4-thiadiazol-5-yl-oxyessigsäureamiden der Formel (I) gemäß Anspruch 1.

8. Verwendung von substituierten 3-Trihalogenmethyl-1,2,4-thiadiazol-5-yl-oxyessigsäureamiden der Formel (I) gemäß Anspruch 1 zur Bekämpfung von Pflanzenwachstum.

9. Verfahren zur Herstellung von herbiziden Mitteln, dadurch gekennzeichnet, daß man substituierte 3-Trihalogenmethyl-1,2,4-thiadiazol-5-yl-oxyessigsäureamide der Formel (I) gemäß Anspruch 1 mit Streckmitteln und/oder oberflächenaktiven Mitteln vermischt.

10. 5-Chlor-3-trihalogenmethyl-1,2,4-thiadiazole der Formeln

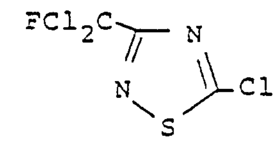

(IIIa)

und

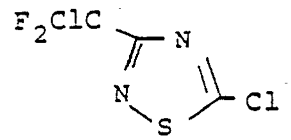

(IIIb)

11. Verfahren zur Herstellung von 5-Chlor-3-trihalogenmethyl-1,2,4-thiadiazolen der Formeln (IIIa) und (IIIb) gemäß Anspruch 9, dadurch gekennzeichnet, daß man 5-Chlor-3-trichlormethyl-1,2,4-thiadiazol der Formel

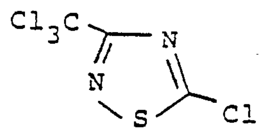

(IV)

mit überschüssigem, wasserfreiem Fluorwasserstoff bei Temperaturen zwischen 100 und 180 °C und bei Drucken bis etwa 50 bar umsetzt und die Produkte durch fraktionierte Destillation isoliert.

**Claims**

1. Substituted 3-trihalogenomethyl-1,2,4-thiadiazol-5-yl-oxyacetic acid amides of the formula

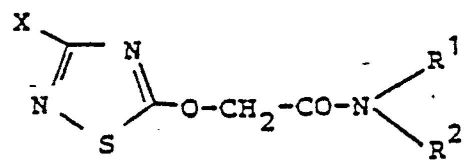

(I)

in which

X represents $—CFCl_2$, $—CF_2Cl$ or $—CF_3$ and

$R^1$ and $R^2$ are identical or different and individually represent alkyl, cyanoalkyl, alkoxyalkyl, alkenyl, alkinyl, alkoxy, each with up to 10 C atoms, cycloalkyl or cycloalkenyl, each with up to 12 C atoms, aralkyl with 1 or 2 C atoms in the alkyl part and 6 or 10 C atoms in the aryl part, which is optionally substituted by halogen, or aryl with 6 or 10 C atoms, it being possible for the aryl radical to be substituted by 1 to 3 halogen atoms, 1 to 3 alkyl or halogenoalkyl groups each with 1 to 4 C atoms, nitro, cyano or alkoxy with 1 to 4 C atoms, or wherein the radicals $R^1$ and $R^2$, together with the nitrogen atom to which they are bonded, form a partially unsaturated and/or benzo-fused monocyclic or bicyclic structure which has up to 15 C atoms and is optionally substituted by 1 to 3 alkyl groups each with 1 to 5 C atoms, or wherein the radicals $R^1$ and $R^2$, together with the nitrogen atom to which they are bonded, form a saturated monocyclic structure which has up to 6 C atoms, is optionally substituted by 1 to 3 alkyl groups each with 1 to 5 C atoms and optionally contains a further nitrogen atom, oxygen atom or sulphur atom.

2. Compounds of the formula (I) according to Claim 1, characterised in that

$R^1$ represents $C_1$-$C_5$-alkyl, cyanoethyl, $C_1$-$C_4$-alkoxy-ethyl, allyl, propargyl, 1-methyl-propargyl or 1,1-dimethyl-propargyl and

$R^2$ represents $C_1$-$C_5$-alkyl, $C_1$-$C_5$-alkoxy, cyanoethyl, $C_1$-$C_4$-alkoxy-ethyl, allyl, propargyl, 1-methyl-propargyl, 1,1-dimethylpropargyl, cyclopentyl, cyclohexyl, 3,4,6-trimethyl-cyclohexen-1-yl, benzyl, naphthyl or phenyl, which is optionally substituted by 1 to 3 radicals (methyl, chlorine, fluorine, trifluoromethyl, methoxy, methylthio, trifluoromethoxy and/or trifluoromethylthio),

or wherein the radicals $R^1$ and $R^2$, together with the nitrogen atom to which they bonded, represent pyrrolidyl, monoalkyl- or dialkyl-pyrrolidyl with 1 to 3 carbon atoms per alkyl group, morpholinyl or dialkylmorpholinyl with 1 to 3 carbon atoms per alkyl group, piperidyl, monoalkyl-, dialkyl- or trialkylpiperidyl with 1 to 3 carbon atoms per alkyl group, perhydroazepinyl (hexamethyleneimino radical), the heptamethyleneimino radical, 1,2,3,4-tetrahydroindolyl, monoalkyl- or dialkyl-tetrahydroindolyl with up to 3 carbon atoms per alkyl group, perhydroindolyl, monoalkyl- or dialkyl-perhydroindolyl with 1 to 3 carbon atoms per alkyl group, 1,2,3,4-tetrahydroquinolyl or 1,2,3,4-tetrahydro-iso-quinolyl, monoalkyl- or

dialkyl-1,2,3,4-tetrahydroquinolyl or -iso-quinolyl with 1 to 3 carbon atoms per alkyl group, perhydroquinolyl or perhydro-iso-quinolyl, monoalkyl- or dialkyl-perhydroquinolyl or -perhydroisoquinolyl with 1 to 3 carbon atoms per alkyl group.

3. (3-Fluorodichloromethyl-1,2,4-thiadiazol-5-yl)-oxyacetic acid N-methylanilide of the formula

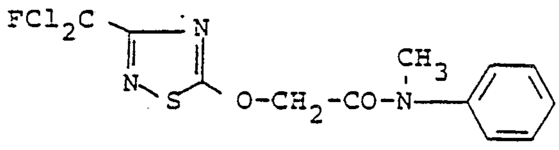

according to Claim 1.

4. (3-Trifluoromethyl-1,2,4-thiadiazol-5-yl)-oxyacetic acid diethylamide of the formula

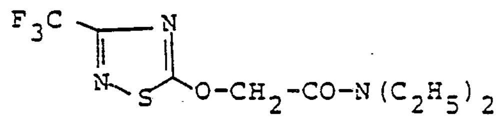

according to Claim 1.

5. (3-Trifluoromethyl-1,2,4-thiadiazol-5-yl)-oxyacetic acid N-ethyl-N-(2.2.2-trifluoroethyl)-amide, (3-fluorodichloromethyl-1,2,4-thiadiazol-5-yl)-oxyacetic acid N-ethyl-N-(2.2.2-trifluoroethyl)-amide and (3-difluorochloromethyl)-1,2,4-thiadiazol-5-yl)-oxyacetic acid N-ethyl-N-(2.2.2-trifluoroethyl)-amide.

6. Process for the preparation of substituted 3-trihalogenomethyl-1,2,4-thiadiazol-5-yl-oxyacetic acid amides of the formula (I) according to Claim 1, characterised in that hydroxyacetic acid amides of the formula

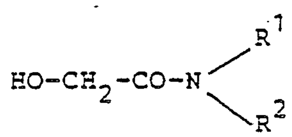

(II)

in which $R^1$ and $R^2$ have the abovementioned meaning, are reacted with a fluorine-containing 5-chloro-3-trihalogenomethyl-1,2,4-thiadiazole of the formula

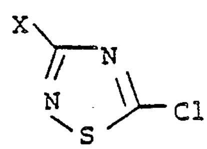

(III)

in which X has the abovementioned meaning, in the presence of an acid acceptor and if appropriate using a diluent.

7. Herbicidal agents, characterised in that they contain substituted 3-trihalogenomethyl-1,2,4-thiadiazol-5-yl-oxyacetic acid amides of the formula (I) according to Claim 1.

8. Use of substituted 3-trihalogenomethyl-1,2,4-thiadiazol-5-yl-oxyacetic acid amides of the formula (I) according to Claim 1 for combating plant growth.

9. Process for the preparation of herbicidal agents, characterised in that substituted 3-trihalogenomethyl-1,2,4-thiadiazol-5-yl-oxyacetic acid amides of the formula (I) according to Claim 1 are mixed with extenders and/or surface-active agents.

10. 5-Chloro-3-trihalogenomethyl-1,2,4-thiadiazoles of the formulae

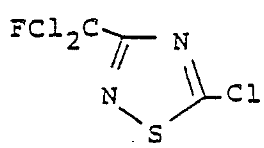

(IIIa)

and

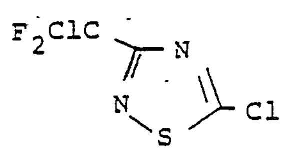

(IIIb)

11. Process for the preparation of 5-chloro-3-trihalogenomethyl-1,2,4-thiadiazoles of the formulae (IIIa) and (IIIb) according to Claim 9, characterised in that 5-chloro-3-trichloromethyl-1,2,4-thiadiazole of the formula

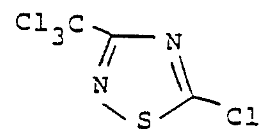

(IV)

is reacted with excess, anhydrous hydrogen fluoride at temperatures between 100 and 180 °C and under pressures of up to about 50 bars and the products are isolated by fractional distillation.

**Revendications**

1. Amides d'acides 3-trihalogénométhyl-1,2,4-thiadiazole-5-yl-oxyacétiques substitués de formule

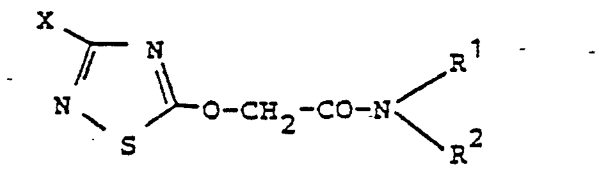

(I)

dans laquelle

X est un groupe $—CFCl_2$, $—CF_2Cl$ ou $—CF_3$ et

$R^1$ et $R^2$ sont identiques ou différents et représentent individuellement un groupe alkyle, cyanalkyle, alkoxyalkyle, alcényle, alcynyle, alkoxy, chacun ayant jusqu'à 10 atomes de carbone, cycloalkyle ou cycloalcényle ayant chacun jusqu'à 12 atomes de carbone, aralkyle ayant 1 ou 2 atomes de carbone dans la partie alkyle et 6 ou 10 atomes de carbone dans la partie aryle, qui est éventuellement substituée par un halogène, ou bien un reste aryle ayant 6 ou 10 atomes de carbone, le reste aryle pouvant être substitué par 1 à 3 atomes d'halogènes, 1 à 3 groupes alkyle ou halogénalkyle ayant chacun 1 à 4 atomes de carbone, nitro, cyano ou alkoxy ayant 1 à 4 atomes de carbone, et les restes $R^1$ et $R^2$ forment conjointement avec l'atome d'azote auquel ils sont liés, un noyau monocyclique ou un noyau bicyclique éventuellement substitué par 1 à 3 groupes alkyle ayant chacun 1 à 5 atomes de carbone, partiellement insaturé et/ou condensé à du benzène, avec jusqu'à 15 atomes de carbone, ou bien les restes $R^1$ et $R^2$ forment, conjointement avec l'atome d'azote auquel ils sont liés, un noyau monocyclique ayant jusqu'à 6 atomes de carbone, éventuellement substitué par 1 à 3 groupes alkyle ayant chacun 1 à 5 atomes de carbone, saturé et contenant, le cas échéant, un autre atome d'azote, un atome d'oxygène ou un atome de soufre.

2. Composés de formule (I) suivant la revendication 1, caractérisés en ce que

$R^1$ est un groupe alkyle en $C_1$ à $C_5$, cyanéthyle, (alkoxy en $C_1$ à $C_4$)-éthyle, allyle, propargyle, 1-méthylpropargyle ou 1,1-diméthylpropargyle et

$R^2$ est un groupe alkyle en $C_1$ à $C_5$, alkoxy en $C_1$ à $C_5$, cyanéthyle, (alkoxy en $C_1$ à $C_4$)-éthyle, allyle, propargyle, 1-méthylpropargyle, 1,1-diméthylpropargyle, cyclopentyle, cyclohexyle, 3,4,6-triméthylcyclohexène-1-yle, benzyle, naphtyle ou phényle qui est éventuellement substitué par 1 à 3 restes (méthyle, chloro, fluoro, trifluorométhyle, méthoxy, méthylthio, trifluorométhoxy et/ou trifluorométhylthio),

ou bien les restes $R^1$ et $R^2$ forment, conjointement avec l'atome d'azote auquel ils sont liés, un reste pyrrolidyle, monoalkyl- ou dialkyl-pyrrolidyle ayant 1 à 3 atomes de carbone par groupe alkyle, morpholinyle ou dialkylmorpholinyle ayant 1 à 3 atomes de carbone par groupe alkyle, pipéridyle, monoalkyl-, dialkyl- ou trialkyl-pipéridyle ayant 1 à 3 atomes de carbone par groupe alkyle, un reste perhydroazépinyle (reste hexaméthylèneimino), le reste heptaméthylèneimino, le reste 1,2,3,4-tétrahydroindolyle, monoalkyl- ou dialkyl-tétrahydroindolyle ayant jusqu'à 3 atomes de carbone par groupe alkyle, un reste perhydroindolyle, monoalkyl- ou dialkyl-perhydroindolyle ayant 1 à 3 atomes de carbone par groupe alkyle, un reste 1,2,3,4-tétrahydroquinolyle ou 1,2,3,4-tétrahydroisoquinolyle, monoalkyl- ou dialkyl-1,2,3,4-tétrahydroquinolyle ou -isoquinolyle ayant 1 à 3 atomes de carbone par groupe alkyle, un reste perhydroquinolyle ou perhydroisoquinolyle, monoalkyl- ou dialkyl-perhydroquinolyle ou -perhydroisoquinolyle ayant 1 à 3 atomes de carbone par groupe alkyle.

3. Le N-méthylanilide d'acide (3-fluorodichlorométhyl-1,2,4-thiadiazole-5-yl)-oxyacétique de formule

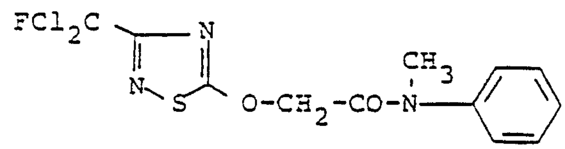

suivant la revendication 1.

4. Le diéthylamide d'acide (3-trifluorométhyl-1,2,4-thiadiazole-5-yl)-oxyacétique de formule

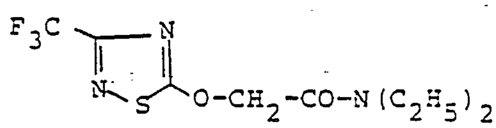

suivant la revendication 1.

5. Le N-éthyl-N-(2.2.2-trifluoréthyl)-amide d'acide (3-trifluorométhyl-1,2,4-thiadiazole-5-yl)-oxyacétique, le N-éthyl-N-(2.2.2-trifluoréthyl)-amide d'acide (3-fluorodichlorométhyl-1,2,4-thiadiazole-5-yl)-oxyacétique et le N-éthyl-N-(2.2.2-trifluoréthyl)-amide d'acide (3-difluorochlorométhyl-1,2,4-thiadiazole-5-yl)-oxyacétique.

6. Procédé de production d'amides d'acides 3-trihalogénométhyl-1,2,4-thiadiazole-5-yl-oxyacétiques substitués de formule (I) suivant la revendication 1, caractérisé en ce qu'on fait réagir des amides d'acide hydroxyacétique de formule

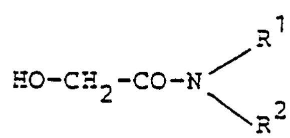

(II)

dans laquelle $R^1$ et $R^2$ ont la définition indiquée ci-dessus, avec un 5-chloro-3-trihalogénométhyl-1,2,4-thiadiazole fluoré de formule

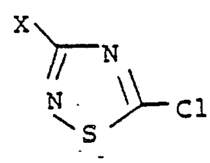

(III)

dans laquelle X a la définition indiquée ci-dessus, en présence d'un accepteur d'acide et en utilisant éventuellement un diluant.

7. Compositions herbicides, caractérisées par une teneur en amides d'acides 3-trihalogénométhyl-1,2,4-thiadiazole-5-yl-oxyacétiques substitués de formule (I) suivant la revendication 1.

8. Utilisation d'amides d'acides 3-trihalogénométhyl-1,2,4-thiadiazole-5-yl-oxyacétiques substitués de formule (I) suivant la revendication 1 pour combattre la croissance de plantes.

9. Procédé de préparation de compositions herbicides, caractérisé en ce qu'on mélange des amides d'acides 3-trihalogénométhyl-1,2,4-thiadiazole-5-yl-oxyacétiques substitués de formule (I) suivant la revendication 1 avec des diluants et/ou des agents tensio-actifs.

10. 5-chloro-3-trihalogénométhyl-1,2,4-thiadiazole de formules

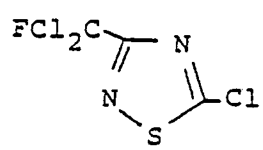

(IIIa)

et

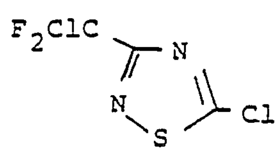

(IIIb)

11. Procédé de production de 5-chloro-3-trihalogénométhyl-1,2,4-thiadiazoles de formules (IIIa) et (IIIb) suivant la revendication 9, caractérisé en ce qu'on fait réagir le 5-chloro-3-trichlorométhyl-1,2,4-thiadiazole de formule

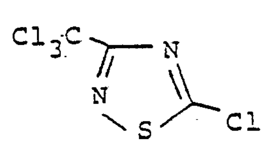

(IV)

avec du fluorure d'hydrogène anhydre en excès à des températures comprises entre 100 et 180 °C et à des pressions allant jusqu'à environ 50 bars et on isole les produits par distillation fractionnée.